# EUROPÄISCHE PATENTSCHRIFT

(11) **EP 0 629 636 B1**
(45) Veröffentlichungstag und Bekanntmachung des Hinweises auf die Patenterteilung: **16.12.1998**
(21) Anmeldenummer: 94108443.6
(22) Anmeldetag: 01.06.1994
(51) Int. Cl.: C07K 7/60, C12P 21/02, A61K 38/08, C12N 1/20

(54) **Lipopeptide aus Actinoplanes sp. mit pharmakologischer Wirkung, Verfahren zu ihrer Herstellung und Verwendung derselben**
Lipopeptides from actinoplanes sp. endowed with pharmacological activity, process for their preparation and their use
Lipopeptides dérivés d'actinoplanes sp. douées d'activité pharmacologique, procédé pour leur préparation et leur emploi

(30) Priorität: 08.06.1993 DE 4319007
(43) Veröffentlichungstag der Anmeldung: 21.12.1994
(60) Teilanmeldung: 98110484.7
(73) Patentinhaber: HOECHST AKTIENGESELLSCHAFT, 65929 Frankfurt am Main (DE)
(72) Erfinder: Hammann, Peter, Dr., D-64832 Babenhausen (DE); Meiwes, Johannes, Dr., D-65510 Idstein (DE); Seibert, Gerhard, Prof. Dr., D-64291 Darmstadt-Arheiligen (DE); Vertesy, Lászlo, Dr., D-65817 Eppstein (DE); Wink, Joachim, Dr., D-63322 Rödermark (DE); Markus, Astrid, Dr., D-65835 Liederbach (DE)

(56) Entgegenhaltungen:
- US-A- 4 001 397
- JOURNAL OF THE AMERICAN CHEMICAL SOCIETY., Bd.95, Nr.7, 4. April 1973, GASTON, PA US Seiten 2352 - 2357 M BODANSZKY ET AL. 'Structure of the peptide antibiotic amphomycin'
- CHEMICAL ABSTRACTS, vol. 73, no. 21, 23. November 1970, Columbus, Ohio, US; abstract no. 109225k, H NAKAGAWA ET AL. 'Novel fatty acid from laspartomycin' Seite 324 ; & JOURNAL OF ANTIBIOTICS., Bd.23, Nr.8, 1970, TOKYO JP Seiten 423 - 424
- CHEMICAL ABSTRACTS, vol. 70, no. 11, 17. März 1969, Columbus, Ohio, US; abstract no. 47853r, J SHOJI ET AL. 'Tsushimycin. I. Isolation and characterization of an acidic acylpeptide containing a new fatty acid' Seite 386 ; & JOURNAL OF ANTIBIOTICS., TOKYO JP
- CHEMICAL ABSTRACTS, vol. 61, no. 6, 14. September 1964, Columbus, Ohio, US; abstract no. 6913, W K HAUSMANN ET AL. 'Structure determination of fatty acids from the antibiotic aspartocin' & ANTIMICROBIAL AGENTS CHEMOTHERAPY, 1963 Seiten 352 - 359
- CHEMICAL ABSTRACTS, vol. 53, no. 2, 25. Januar 1960, Columbus, Ohio, US; abstract no. 11511, Y HINUMA 'Zaomycin, a new antibiotic from a Streptomyces sp.' & JOURNAL OF ANTIBIOTICS, SER. A, Nr.7, 1954, TOKYO JP Seiten 134 - 136
- CHEMICAL ABSTRACTS, vol. 53, no. 2, 25. Januar 1960, Columbus, Ohio, US; abstract no. 8285, G F GAUZE ET AL. 'Crystallomycin, a new antibiotic' & ANTIBIOTIKI, Bd.2, Nr.6, 1957 Seiten 9 - 14

## Beschreibung

Die Erfindung betrifft Lipopeptide mit sehr homologen Aminosäuresequenzen, jedoch unterschiedlichen Fettsäureresten (Lipidanteil), die von Actinoplanes sp. während der Fermentation synthetisiert und in das Kulturmedium abgegeben werden, ein Verfahren zur Isolierung der Lipopeptide aus dem Kulturmedium und ihre Aufreinigung, die Verwendung der Lipopeptide als pharmakologische Wirkstoffe, insbesondere gegen gram-positive Bakterien, sowie Actinoplanes sp. DSM 7358 zur Herstellung der o. g. Lipopeptide.

Sekundärmetabolite aus Mikroorganismen werden erfolgreich zur Behandlung von Infektionskrankheiten eingesetzt. Bei Sekundärmetaboliten handelt es sich um niedermolekulare Verbindungen, deren Bildung in "biosynthetischen Einbahnstraßen", die vom Primärmetabolismus abzweigen, erfolgt und deren Funktion für den jeweiligen Produzenten ungeklärt ist. Bis heute sind ca. 8000 aus Kulturen verschiedener Mikroorganismen (vor allem Pilze und Bakterien der Gattung Streptomyces) isolierte Sekundärmetabolite bekannt.

Haupteinsatzgebiet dieser Sekundärmetabolite ist die Therapie von Infektionskrankheiten. Durch den breiten Einsatz kommt es jedoch häufig zur Resistenzbildung, so daß ein ständiger Bedarf an neuen Antibiotika und Wirkstoffen mit neuen Wirkmechanismen besteht (Neu H.C., Science 257, 1992, S. 1064-1073).

Daneben hat sich das Indikationsgebiet mikrobieller Wirkstoffe auch auf Krankheiten, die nicht zu den Infektionskrankheiten zählen (z.B. Tumortherapie, Immunmodulation oder zur Regulation des Fettstoffwechsels) und auf den Pflanzenschutz (Herbizide und Insektizide) ausgeweitet. Die eingesetzten Wirkstoffe sind allerdings oft noch mit Mängeln behaftet, gekennzeichnet durch unbefriedigende Wirkhöhen, eine zu hohe Toxizität und/oder unerwünschte Nebenwirkungen.

In der Literatur sind Lipopeptide beschrieben, deren Aminosäureanteil in bezug auf die Sequenz identisch mit oder in bezug auf die Aminosäurezusammensetzung gleich oder sehr ähnlich den erfindungsgemäßen Lipopeptiden ist. Diese Lipopeptide unterscheiden sich jedoch grundsätzlich von den erfindungsgemäßen Lipopeptiden im Lipidanteil.

Beispiele für Lipopeptide, wie oben angesprochen, sind:
- Amphomycin Antibiot. [J. Antibiotics, 38, S. 517 (1965)];
- Glumamycin [J. Antibiotics, 38, S. 517 (1965)];
- Zaomycin [J. Antibiot. Ann., S. 194 (1960)];
- Aspartocin [Antibiot. Ann., 194 (1960)];
- Tsuhimycin [J. Antibiotics, 21, S. 439 (1968)];
- Laspartomycin [J. Antibiotics 21, S. 55 (1968)].

Diese Lipopeptide, welche als Amphomycin-Typ Lipopeptide bezeichnet werden, werden von Mikroorganismen der Gattung Streptomyces synthetisiert. Ihre antibiotische Wirksamkeit entfalten sie gegen gram-positive Bakterien, wie z.B. Strepto-, Staphylo- und Enterococcen. Insbesondere Stämme der Gattungen Staphylo- und Enterococcus erweisen sich in letzter Zeit zunehmend als Problemkeime. Unter Problemkeimen versteht der Fachmann solche Mikroorganismen, deren effektive Bekämpfung inzwischen aufgrund von Resistenzen mit gängigen Antibiotika (z.B. β-Lactamantibiotika oder Glykopeptid-Antibiotika, wie beispielsweise Vancomycin oder Teikoplanin) nicht möglich ist.

Eine Gruppe von Mikroorganismenstämmen, die Resistenzen ausgebildet haben, sind z.B. die Methicillin-resistenten Staphylococcus aureus-Stämme, kurz MRSA-Stämme genannt. Es ist inzwischen bekannt, daß diese MRSA-Stämme häufig nicht nur gegen Methicillin sondern außerdem gegen weitere Antibiotika (z.B. Vancomycin) Resistenzen ausgebildet haben.

Abgesehen von den schon genannten Verbindungen aus Streptomyceten ist eine Verbindung aus Actinoplanes nipponensis ATCC 31145 bekannt, die aufgrund ihres Wirkspektrums und der beschriebenen physikalisch-chemischen Eigenschaften strukturelle Ähnlichkeiten mit den erfindungsgemäßen Lipopeptiden aufweist und Compound 41.012 genannt wird (US Patent 4,000,397). Die Fermentation von Actinoplanes nipponensis ATCC 31145 unter verschiedenen Kulturbedingungen führt immer zu relativ geringen Ausbeuten an Compound 41.012.

Aufgabe der Erfindung ist es, mikrobielle Naturstoffe mit verbesserten Eigenschaften bereitzustellen.

Diese Aufgabe wird erfindungsgemäß gelöst durch Fermentation von Actinoplanes sp. in einer Nährlösung mit Kohlenstoff- und Stickstoffquelle sowie den üblichen anorganischen Salzen, bis sich Lipopeptide, vorzugsweise die nachfolgend näher bezeichneten Lipopeptide, in dem Kulturmedium anhäufen, anschließender Isolierung der Lipopeptide aus dem Kulturmedium und die Trennung des Gemisches in seine Einzelkomponenten. Die isolierten Lipopeptide besitzen pharmakologische Aktivität und damit therapeutische Wirksamkeit und können als Antibiotika mit Wirkung gegen gram-positive Bakterien, vorzugsweise gegen Glycopeptid-resistente Stämme, eingesetzt werden.

Die Erfindung betrifft somit:
1. Lipopeptide der Formel I, 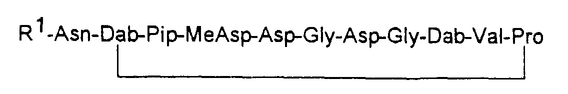
R1-Asn-Dab-Pip-MeAsp-Asp-Gly-Asp-Gly-Dab-Val-Pro
 in der
   - R¹: eine einfach oder mehrfach ungesättigte, gesättigte, oder unabhängig davon eine verzweigte oder unverzweigte Fettsäure oder Hydroxyfettsäure mit einer Kettenlänge von 6 bis einschließlich 22 Kohlenstoffatomen bedeutet.
2. Insbesondere Lipopeptide der Formel I, dadurch gekennzeichnet, daß R¹ eine
   a) iC₁₃-Fettsäure,
   b) iC₁₄-Fettsäure,
   c) aiC₁₃-Fettsäure oder
   d) aiC₁₅-Fettsäure bedeutet.
3. Besonders bevorzugt Lipopeptide der Formel I, worin
   R₁ = 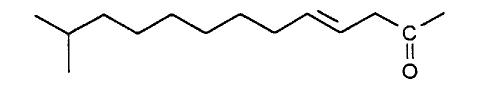 oder
   R₁ = 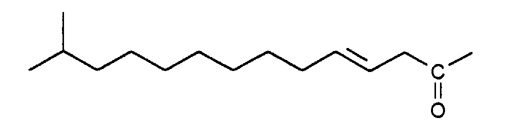 oder
   R₁ = 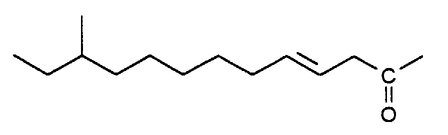 oder
   R₁ = 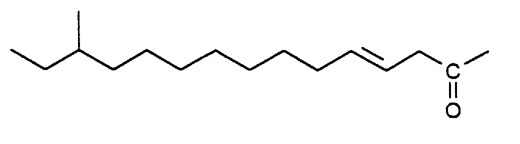 bedeutet.
4. Ein Verfahren zur Herstellung eines oder mehrerer Lipopeptide der Formel I, wie unter 1. charaktisiert, eines oder mehrerer Lipopeptide, wie unter 2. charakterisiert, oder eines oder mehrerer Lipopeptide der Formel II, wie unter 3. charakterisiert, dadurch gekennzeichnet, daß man Actinoplanes sp. in einem Kulturmedium fermentiert, bis sich ein oder mehrere Lipopeptide in dem Kulturmedium anhäufen und man ein oder mehrere Lipopeptide der Formel I aus dem Kulturmedium aufreinigt.
5. Eine Verwendung eines Lipopeptids der Formel 1 zur Herstellung eines Antibiotikums gegen gram-positive Bakterien, besonders bevorzugt gegen Glycopeptid-resistente Bakterien.
6. Actinoplanes sp. DSM 7358.

Im folgenden wird die Erfindung detailliert beschrieben, insbesondere in ihren bevorzugten Ausführungsformen. Ferner wird die Erfindung durch den Inhalt der Patentansprüche bestimmt.

Definition von Begriffen:
- Dab bedeutet 2,3-Diamino-buttersäure;
- Pip bedeutet Pipecolinsäure (Synonym = Homoprolin);
- MeAsp bedeutet β-Methylaspartat;
- Gly bedeutet Glycin;
- Asn bedeutet Asparagin;
- Asp bedeutet Asparaginsäure;
- Val bedeutet Valin;
- Pro bedeutet Prolin;
- n bedeutet normal/unverzweigt und
- CID bedeutet "collisional induced decay".

Die Abkürzung "i" steht für "iso", während "ai" die Bedeutung "ante-iso" hat. Diese Bezeichnungen sind dem Fachmann in bezug auf Fettsäuren bekannt (Biochemistry, Zubay, Verlag Addison Wesley in London, Amsterdam, 1983).

Alle Prozentangaben beziehen sich, wenn nichts anderes angegeben ist, auf das Gewicht. Mischungsverhältnisse bei Flüssigkeiten beziehen sich auf das Volumen, wenn keine anderen Angaben gemacht werden.

Das erfindungsgemäße Verfahren kann für die Fermentation im Labormaßstab (Milliliter- bis Literbereich) und für den industriellen Maßstab (Kubikmetermaßstab) eingesetzt werden.

Actinoplanes sp. wird aus einer Erdprobe isoliert. Für die Aufreinigung aus der Erde wird diese unter Herstellung einer Verdünnungsreihe mit einer physiologischen NaCl-Lösung (0,9 %) aufgeschwemmt. Die verschiedenen Verdünnungen (10⁰-10⁶) werden anschließend auf Actinomycetennährboden ausplattiert. Nach einer Bebrütung der Kulturen bei 30°C für die Dauer von 2 bis 14 Tagen entstehen Actinomycetenkolonien, die vereinzelt und durch mehrere, aufeinanderfolgende Aufreingungsschritte isoliert werden können.

Die Bestimmung der Gattungen wird anhand von morphologischen und taxonomischen Kriterien nach dem Fachmann bekannten Methoden vorgenommen. Charakteristisch für die Gattung Actinoplanes sind vor allem die beweglichen Sporen.

Aufgrund von aufeinanderfolgenden Isolierungs- und Aufreinigungsschritten kann man von Actinoplanes sp. eine Kolonie isolieren, die ein oder mehrere Verbindungen der Lipopeptide, bevorzugt die Lipopeptide A 1437 A, B, C, D, E, F, G, H, K, L und/oder M sehr effizient in das Kulturmedium abgibt und als Hauptproduzent bezeichnet wird.

Als Hauptproduzent wird ein Isolat bezeichnet, das ein oder mehrere Verbindungen der erfindungsgemäßen Lipopeptide in einer 10- bis 100-fach erhöhten Menge im Vergleich zu Isolaten der gleichen Actinoplanesart produziert bzw. in das Kulturmedium abgibt.

Eine stark produzierende Kolonie von Actinoplanes sp. wird vermehrt. Ein Isolat wurde bei der Deutschen Sammlung von Mikroorganismen und Zellkulturen GmbH, Mascheroder Weg 1B, 3300 Braunschweig, Deutschland, nach den Regeln des Budapester Vertrages am 18. Juni 1990 unter der folgenden Nummer hinterlegt: Actinoplanes sp. DSM 7358.

Actinoplanes sp. DSM 7358 besitzt ein orangefarbenes Mycel und ist durch globose Sporangien charakterisiert.

In einer Nährlösung (auch als Kulturmedium bezeichnet), die eine Kohlenstoffquelle und eine Stickstoffquelle sowie die üblichen anorganischen Salze enthält, produziert Actinoplanes sp., bevorzugt DSM 7358, ein oder mehrere Verbindungen der erfindungsgemäßen Lipopeptide.

Anstelle des Stammes DSM 7358 können auch dessen Mutanten und Varianten eingesetzt werden, die ein oder mehrere Verbindungen der erfindungsgemäßen Lipopeptide synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Das Screening nach Mutanten und Varianten, die ein oder mehrere Verbindungen der erfindungsgemäßen Lipopeptide synthetisieren, erfolgt nach folgendem Schema:
- Abtrennung des Mycels nach der Fermentation;
- Fällung der Lipopeptide bei pH 1 bis 2 (4°C);
- Aufnehmen des Niederschlages in H₂O/MeOH (1:1);
- Analytik mittels HPLC, DC oder Hemmhoftest.

Die im folgenden beschriebenen Fermentationsbedingungen gelten für Actinoplanes sp., das hinterlegte Isolat DSM 7358 sowie Mutanten und Varianten von diesen.

In einer Nährlösung, die eine Kohlenstoffquelle und eine Stickstoffquelle sowie die üblichen anorganischen Salze enthält, produziert Actinoplanes sp., vorzugsweise DSM 7358, bevorzugt die Lipopeptide A 1437 A-H sowie K, L, M.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie z.B. Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Lipopeptide verläuft besonders gut in einer Nährlösung, die etwa 0,1 bis 5 %, bevorzugt 0,3 bis 2 % Fleischextrakt und 0,2 bis 5 %, bevorzugt 0,5 bis 2 % Saccharose und 0,05 bis 5 g/l, bevorzugt 0,1 bis 0,5 g/l Hefeextrakt und 0,05 bis 2 g/l, bevorzugt 0,1 bis 1 g/l Magnesiumsulfat und 0,05 bis 10 g/l, bevorzugt 0,1 bis 1 g/l Kalium- oder Natriumdihydrogen-phosphat und 0 bis 100 µM, bevorzugt 5 bis 20 µM Eisen-III-Chlorid enthalten. Die Angaben in Prozent sind jeweils bezogen auf das Gewicht der gesamten Nährlösung.

In dieser Nährlösung bildet Actinoplanes sp., vorzugsweise Actinoplanes DSM 7358 ein Gemisch aus Lipopeptiden. Vorzugsweise besteht das Gemisch aus 11 unterschiedlichen, nachweisbaren Lipopeptiden. Davon sind Lipopeptide gemäß der vorliegenden Erfindung die nachfolgend charakterisierten Lipopeptide der Formel I, die A 1437 C, D, F und H genannt wurden.

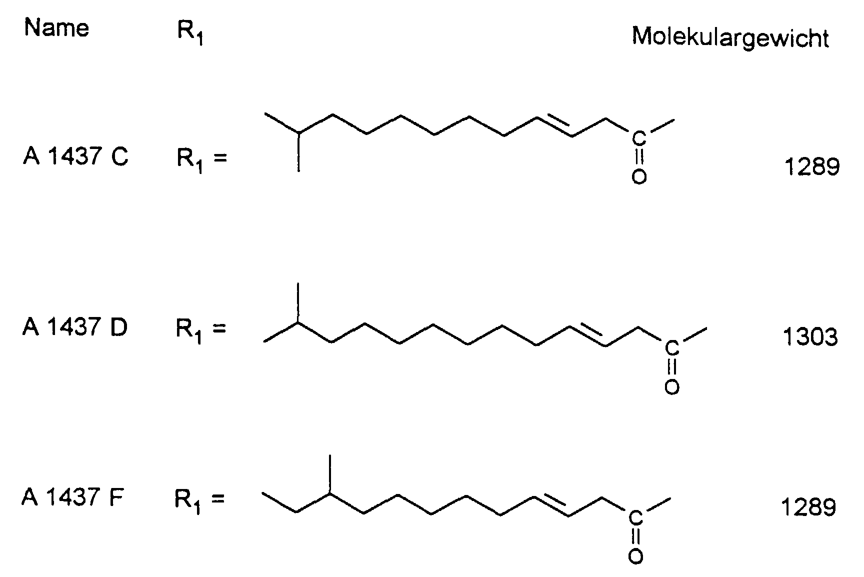

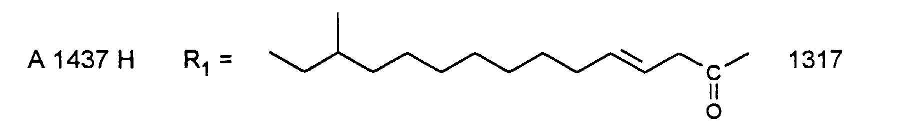

Lipopeptid A 1437 C besitzt eine bisher in Lipopeptiden vom Amphomycin-Typ noch nicht bekannte iso-C₁₃-Fettsäure. Die Aminosäurezusammensetzung und -sequenz von A 1437 C ist aus anderen Lipopeptiden nicht bekannt.

A 1437 D besitzt eine Fettsäure des Typs iso-C₁₄ und damit die aus dem Tsushimycin (Shoji et al., The Jounal of Antibiotics 21, 439 (1968)) bekannte Fettsäure. A 1437 D unterscheidet sich jedoch in seiner Aminosäurezusammensetzung und -sequenz von den bisher aus dem Stand der Technik bekannten Lipopeptiden. Die Aminosäurezusammensetzung von Amphomycin, Glumamycin, Zaomycin und Tsushimycin ist gemäß der Literatur identisch (Strong et al., Antimicrobial Agents and Chemotherapy 1970, 42; Bodanszley et al., J. Am. Chem. Soc. 95, 2352 (1973), Inoue, Bull. Chem. Soc. Jap. 35, 1556 (1962)).

A 1437 F besteht aus einer ante-iso-C₁₃-Fettsäure, besitzt also damit den gleichen Fettsäuretyp wie aus dem Amphomycin bekannt. Seine Aminosäuresequenz und - zusammensetzung ist aus dem Stand der Technik unbekannt.

Das Lipopeptid A 1437 H hat, ebenso wie Aspartocin, eine ante-iso-C₁₅-Fettsäure. Die Aminosäuresequenz und Zusammensetzung von A 1437 H ist aus dem Stand der Technik unbekannt.

Die Lipopeptide A 1437 C, D, F und H besitzen alle eine Verknüpfung der Carboxyfunktion des C-terminalen Prolins mit der ß-Aminofunktion des aminoterminal lokalisierten Dab. Diese Verknüpfung ist durch " 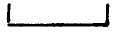 " in Formel I dargestellt.

Neben den Lipopeptiden gemäß der vorliegenden Erfindung bildet Actinoplanes sp., bevorzugt Actinoplanes DSM 7358, die nachfolgend charakterisierten Lipopeptide A, B, E, G, K, L und M der Formel II:

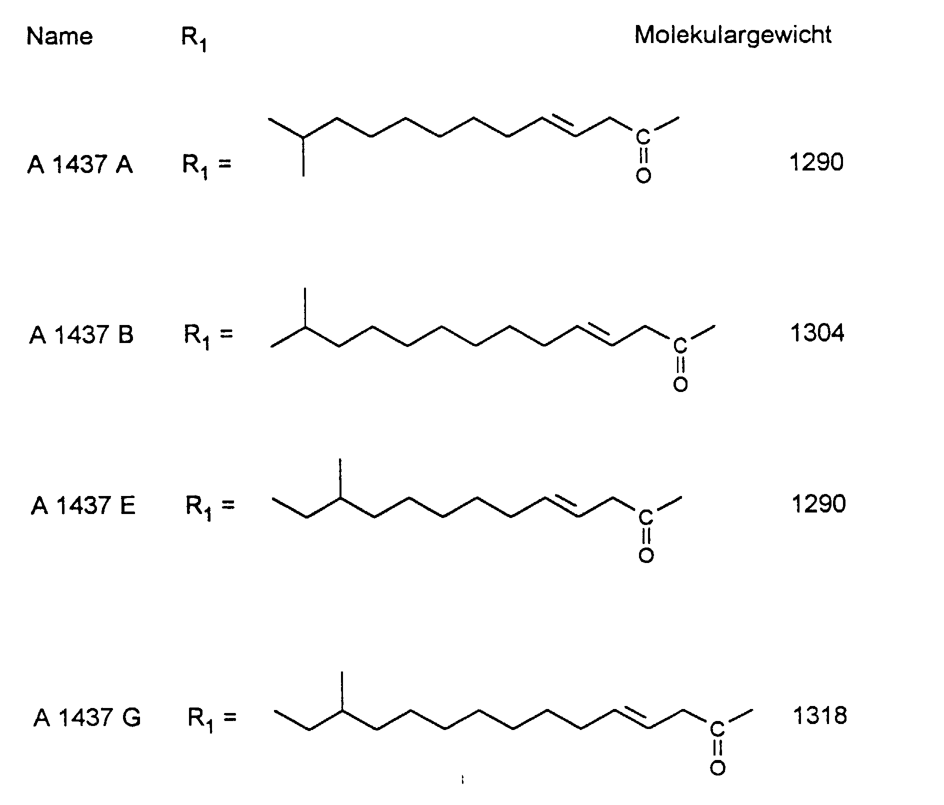

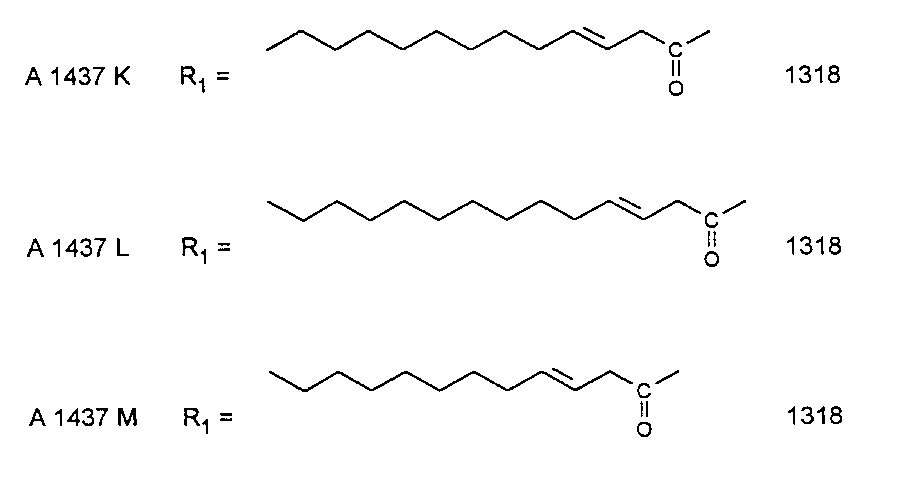

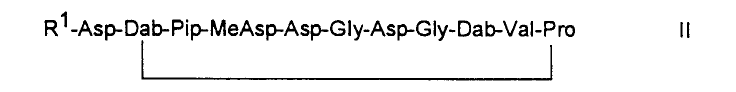
R^1-Asp-Dab-Pip-MeAsp-Asp-Gly-Asp-Gly-Dab-Val-Pro II


Lipopeptid A 1437 A besitzt eine bisher in Lipopeptiden vom Amphomycin-Typ noch nicht bekannte ante-iso-C₁₃-Fettsäure. Die Aminosäuresequenz von A 1437 A entspricht der von Amphomycin, welches jedoch eine ante-iso-C₁₃-Fettsäure besitzt (Bodanszky et al. J. Am. Chem. Soc. 95, 2352 (1973)).

A 1437 B hat eine Fettsäure des Typs iso-C₁₄ und damit die aus dem Tsushimycin (Shoji et al., J. of Antibiotics 21, 439 (1968)) bekannte Fettsäure und besitzt die Aminosäuresequenz des Amphomycins (Bodanszky et al., J. Am. Chem. Soc. 95, 2352 (1973)).

A 1437 E ist mit dem aus Streptomyceten bekannten Amphomycin identisch.

Das Lipopeptid A 1437 G hat ebenso wie Aspartocin (Hausmann et al., Antimicrobial Agents and Chemotherapy 1963, 352) eine ante-iso-C₁₅-Fettsäure. Die Aminosäuresequenz von A 1437 G entspricht der des Amphomycins (Bodansky et al., J. Am. Chem. Soc. 95, 2352 (1973)).

A 1437 K, L und M besitzen unverzweigte Fettsäuren mit einer Kettenlänge von C₁₂-C₁₄. Die Aminosäuresequenz der drei genannten Lipopeptide entspricht der des Amphomycins.

Die Lipopeptide A 1437 A, B, E und G besitzen alle eine Verknüpfung der Carboxyfunktion des C-terminalen Prolins mit der β-Aminofunktion des aminoterminal lokalisierten Dab. Diese Verknüpfung ist durch " 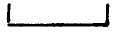 " in Formel II dargestellt.

Je nach Zusammensetzung der Nährlösung kann der mengenmäßige Anteil eines oder mehrerer der erfindungsgemäßen Lipopeptide variieren. Außerdem kann durch die Medienzusammensetzung die Synthese einzelner Lipopeptide gesteuert werden, so daß ein Lipopeptid gar nicht bzw. in einer Menge unterhalb der Nachweisgrenze vom Mikroorganismus hergestellt wird.

Das Kulturmedium von Actinoplanes sp., vorzugsweise DSM 7358, enthält Lipopeptide mit einer einfach oder mehrfach ungesättigten, gesättigten oder unabhängig davon einer verzweigten oder unverzweigten Fettsäure oder Hydroxyfettsäure mit einer Kettenlänge von 6 bis einschließlich 22 Kohlenstoffatomen, bevorzugt von 10 bis einschließlich 20 Kohlenstoffatomen, besonders bevorzugt von 13,14 oder 15 Kohlenstoffatomen.

Derartige Fettsäuren sind dem Fachmann bekannt, so z.B. aus Römpp Chemie Lexikon, Prof. Falbe und Prof. Regitz, 9. Aufl. Georg Thieme Verlag Stuttgart, New York oder aus The Encyclopedia of Chemistry C.A. Hampel and G.G. Hawley, 3. Aufl. Van Nostrand Reinhold Company, New York.

Die folgende Aufzählung von Fettsäuren ist beispielhaft, erhebt keinen Anspruch auf Vollzähligkeit und stellt keine Beschränkung dar.

Gesättigte, unverzweigte Fettsäuren an den erfindungsgemäßen Lipopeptiden sind beispielsweise die Capron-, Önanth-, Capryl-, Pelargon-, Caprin-, Undecan-, Laurin-, Tridecan-, Myristin-, Pentadecan-, Palmitin-, Margarin-, Stearin-, Nonadecan-, Arachin-, Behensäure.

Gesättigte, verzweigte Fettsäuren an den erfindungsgemäßen Lipopeptiden sind beispielsweise die Isobutter- oder die Isovaleriansäure oder die entsprechenden Säuren in der Konfiguration "ante-iso".

Einfach ungesättigte, unverzweigte Fettsäuren an den erfindungsgemäßen Lipopeptiden sind beispielsweise die Acryl- oder die Crotonsäure.

Eine zweifach ungesättigte, unverzweigte Fettsäure an den erfindungsgemäßen Lipopeptiden ist beispielsweise die Sorbinsäure.

Dreifach ungesättigte, unverzweigte Fettsäuren an den erfindungsgemäßen Lipopeptiden sind beispielsweise die Linolen- oder die Elaeostearinsäure.

Eine vierfach ungesättigte, unverzweigte Fettsäure an den erfindungsgemäßen Lipopeptiden ist beispielsweise die Arachidonsäure.

Eine fünffach ungesättigte, unverzweigte Fettsäure an den erfindungsgemäßen Lipopeptiden ist beispielsweise die Clupanodensäure.

Eine sechsfach ungesättigte, unverzweigte Fettsäure an den erfindungsgemäßen Lipopeptiden ist beispielsweise die Docosahexaensäure.

Weiterhin können in dem Kulturmedium Lipopeptide mit mehrfach verzweigten Fettsäuren, wie z.B. die 2',4',6',8'-Tetramethyldecansäure, vorkommen.

Hydroxyfettsäuren an den erfindungsgemäßen Lipopeptiden sind beispielsweise die an 2'- und 3'-Position und/oder am Ende der Kohlenstoffkette hydroxilierten Fettsäuren in der Konfiguration "iso" oder "ante-iso".

Bei Zugabe von 0,01 bis 5 %, vorzugsweise 0,02 bis 0,1 % L-Valin zu der oben beschriebenen Nährlösung bildet der Stamm Actinoplanes sp. bevorzugt die Lipopeptide A 1437 B und D. Bei Zugabe von 0,01 bis 5 %, vorzugsweise 0,1 bis 0,5 % L-Leucin zur oben beschriebenen Nährlösung bildet der Stamm Actinoplanes sp. bevorzugt die Lipopeptide A 1437 A und C.
Bei Zugabe von 0,01 bis 5 %, vorzugsweise 0,05 bis 0,5 % L-Isoleucin zur oben beschriebenen Nährlösung bildet der Stamm Actinoplanes sp. vor allem die Lipopeptide A 1437 E, F, G und H. Bei Zugabe von 0,01 bis 5 %, vorzugsweise 0,05 bis 0,5 % L-α-Aminobuttersäure zur oben beschriebenen Nährlösung bildet der Stamm Actinoplanes sp. bevorzugt das Lipopeptid K. Bei Zugabe von 0,01 bis 5 %, vorzugsweise 0,05 bis 0,5 % L-Norvalin zur oben beschriebenen Nährlösung bildet der Stamm Actinoplanes sp. bevorzugt die Lipopeptide L und/oder M. Gleiches gilt für den bevorzugten Stamm DSM 7358.

Neben diesen Aminosäuren können auch die entsprechenden α-Ketosäuren der genannten Aminosäuren (α-Ketoisovalerat, α-Ketoisocaproat, α-Keto-β-methylvalerat, α-Ketovalerat) oder deren korrespondierende Säuren (Isobutyrat, Isovalerat, α-Methylbutyrat, n-Butyrat, Propionat, Valerat) in den entsprechenden Konzentrationen oder weitere Substanzen, die in die Fettsäurebiosynthese eingreifen können, eingesetzt werden. Die Kultivierung der Mikroorganismus erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35°C, vorzugsweise bei etwa 25 bis 35°C, insbesondere bei 28 bis 32°C durchgeführt werden. Der pH-Bereich sollte zwischen 6 und 8 liegen, vorzugsweise zwischen 6,5 und 7,5. Man kultiviert den Mikroorganismus unter diesen Bedingungen im allgemeinen über einen Zeitraum von 24 bis 300 Stunden, vorzugsweise 36 bis 140 Stunden.

Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 72 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Hefe-Malz-Agar oder Nutrient-broth-Agar (Standardmedium für Mikroorganismen mit den Hauptbestandteilen Pepton, Kochsalz und Agar, z.B. von Fa. Difco) wachsen läßt.

Der Fermentationsverlauf kann anhand des pH-Wertes der Kulturen oder des Mycelvolumens sowie durch chromatographische Methoden, wie z.B. Dünnschichtchromatographie oder High Pressure Liquid Chromatography oder Ausprüfen der biologischen Aktivität überwacht werden. Eine erfindungsgemäße Verbindung ist sowohl im Mycel als auch im Kulturfiltrat enthalten, der größte Teil (≥ 90 %) befindet sich jedoch im Kulturfiltrat.

Das im folgenden beschriebene Isolierungsverfahren dient zur Aufreinigung der erfindungsgemäßen Lipopeptide, bevorzugt jedoch für die Lipopeptide A 1437 C, A 1437 D, A 1437 F und A 1437 H.

Die Isolierung bzw. Aufreinigung eines erfindungsgemäßen Lipopeptids aus dem Kulturmedium erfolgt nach bekannten Methoden unter Berücksichtigung der chemischen, physikalischen und biologischen Eigenschaften der Naturstoffe. Zum Testen der Antibiotika-Konzentration im Kulturmedium oder in den einzelnen Isolierungsstufen kann die Dünnschichtchromatographie, beispielsweise an Kieselgel mit Isopropanol/25%igem NH₃ als Laufmittel oder HPLC verwendet werden. Die Detektion bei der dünnschichtchromatographischen Auftrennung kann beispielsweise durch Färbereagentien wie Anisaldehyd erfolgen, wobei die Menge der gebildeten Substanz zweckmäßig mit einer Eichlösung verglichen wird.

Zur Isolierung eines erfindungsgemäßen Lipopeptides wird das Mycel zunächst von der Kulturbrühe nach den üblichen Verfahren abgetrennt und anschließend das Kulturfiltrat, vorzugsweise bei 4°C auf einen pH-Wert von pH 0,5 bis einschließlich pH 4, vorzugsweise von pH 1,5 bis einschließlich pH 2,5 eingestellt. Die Einstellung des pH-Wertes und damit die Fällung der Lipopeptide A 1437 kann mit allen handelsüblichen Säuren erfolgen. Die Lösung wird bis zu 16 Std., vorzugsweise bis zu 4 Std., inkubiert und anschließend der entstandene Niederschlag abzentrifugiert.

Der Niederschlag, der die gesamten Lipopeptide enthält, wird in 1/20 des ursprünglichen Volumens Aqua bidest. resuspendiert und mit NaOH auf pH 6 bis 7 eingestellt. Der Niederschlag geht dabei vollständig in Lösung; die Lösung wird auf -20°C gekühlt und lyophilisiert. Das Lyophilisat, in der Folge als Rohprodukt bezeichnet, enthält 5 bis 30 % Lipopeptide und wird für die weitere Isolierung eingesetzt.

Die weitere Aufreinigung eines oder mehrerer erfindungsgemäßen Lipopeptide erfolgt durch Chromatographie an geeigneten Materialien, vorzugsweise z.B. an Kieselgel, Aluminiumoxid, Ionenaustauschern oder Adsorberharzen und ganz besonders bevorzugt an stark oder schwach basischen Anionenaustauschern. Mit Hilfe dieser Chromatographie werden die Lipopeptide getrennt, die Asp bzw.

Asn als aminoterminal lokalisierte Aminosäure besitzen. Die Chromatographie der Lipopeptide erfolgt mit gepufferten wässrigen Lösungen oder Gemischen von wässrigen und alkoholischen Lösungen.

Unter gepufferten wässrigen Lösungen versteht man z.B. Wasser, Phosphatpuffer, Ammoniumacetat, Citratpuffer, Boratpuffer in einer Konzentration von 0 bis 1 M, vorzugsweise 1 bis 100 mM, besonders bevorzugt werden phosphatgepufferte Lösungen in einer Konzentration von 1 bis 100 mM eingesetzt.

Unter Gemischen von wässrigen oder alkoholischen Lösungen versteht man alle mit Wasser mischbaren organischen Lösemittel, vorzugsweise Methanol, Acetonitril, in einer Konzentration von 10 bis 80 % Lösemittel, vorzugsweise 40 bis 60 % Lösemittel oder auch alle gepufferten wässrigen Lösungen, die mit organischen Lösemitteln mischbar sind. Die zu verwendeten Puffer sind die gleichen wie oben angegeben.

Die Trennung der Lipopeptide aufgrund ihrer unterschiedlichen Fettsäuren erfolgt mit Hilfe der Reversed Phase Chromatographie, beispielsweise an MCI® (Adsorberharz von Mitsubishi, Japan). Besonders bevorzugt ist die Reversed Phase Chromatographie an hydrophoben Materialien, vorzugsweise RP-8- oder RP-18-Phasen Chromatographie. Außerdem kann die Trennung mit Hilfe der Kieselgelchromatographie erfolgen.

Die Chromatographie der Lipopeptide erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Acetonitril verwendet.

Unter gepufferten oder angesäuerten wäßrigen Lösungen versteht man z.B. Wasser, Phosphatpuffer, Ammoniumacetat, Citratpuffer, Boratpuffer in einer Konzentration von 0 bis 0,5 M sowie Ameisensäure, Essigsäure, Trifluoressigsäure oder allen handelsüblichen, dem Fachmann bekannten Säuren, vorzugsweise in einer Konzentration von 0 bis 1 %. Besonders bevorzugt wird 0,1 %.

Chromatographiert wird mit einem Gradienten, der mit 100 % Wasser beginnt und mit 100 % Lösemittel endet, vorzugsweise wird ein linearer Gradient von 40 bis 60 % Acetonitril gefahren.

Die Reihenfolge der beiden vorgenannten Chromatographien (Chromatographie zur Trennung der Lipopeptide nach den Aminosäuren Asp oder Asn und nach dem Fettsäuretypus) ist umkehrbar. Bevorzugt ist die Trennung der Lipopeptide nach unterschiedlichen Aminosäuren im ersten Schritt und nachfolgend erst ihre Trennung nach dem Fettsäuretypus.

Enthält das vorgenannte Rohprodukt Lipopeptide mit einheitlicher Fettsäure, wird die vorbeschriebene Chromatographie (Trennung der Lipopeptide aufgrund unterschiedlicher Fettsäuren) zur Entsalzung und weiteren Aufreinigung der Lipopeptide eingesetzt.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad) oder Fractogel TSK HW 40® (Fa. Merck, Deutschland oder Toso Haas, USA).

Die erfindungsgemäßen Lipopeptide sind im festen Zustand und in Lösungen im pH-Bereich zwischen 4 und 8, insbesondere 5 und 7, stabil und lassen sich damit in übliche galenische Zubereitungen einarbeiten.

Eine oder mehrere Verbindungen der erfindungsgemäßen Lipopeptide eignen sich aufgrund ihrer wertvollen pharmakologischen Eigenschaften zur Anwendung als Arzneimittel.

Die erfindungsgemäßen Substanzen besitzen pharmakologische Wirksamkeit insbesondere als Antibiotikum gegen gram-positive Bakterien, besonders bevorzugt gegen Glycopeptid-resistente Stämme.

Bei Penicillin- bzw. Methicillin-resistenten Stämmen (MRSA-Stämmen), die weitere Resistenzen gegen Antibiotika ausgebildet haben, besitzen häufig nur Glycopeptide wie Vancomycin oder Teicoplanin eine therapeutisch ausreichende Wirkung. Zunehmend treten jedoch auch gegen diese Antibiotika resistente Stämme auf (FEMS Microbiol. Lett. 98 (1992) 109 bis 116). Ein oder mehrere Verbindungen der erfindungsgemäßen Lipopeptide besitzen eine hervorragende Wirkung auch gegen diese Problemkeime.

Die Erfindung betrifft auch pharmazeutische Zubereitungen einer oder mehrerer Verbindungen der erfindungsgemäßen Lipopeptide.

Ein oder mehrere Verbindungen der erfindungsgemäßen Lipopeptide, bevorzugt ein oder mehrere Verbindungen der Lipopeptide A 1437 C, D, F und H, können grundsätzlich als solche in Substanz verabreicht werden. Bevorzugt ist die Verwendung in Mischung mit geeigneten Hilfsstoffen oder Trägermaterial. Als Trägermaterial können bei Tierarzneimitteln die üblichen Futtermittelmischungen bzw. beim Menschen alle pharmakologisch verträglichen Trägermaterialien und/oder Hilfsstoffe verwendet werden.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Micheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Lipopeptide enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere Verbindungen der erfindungsgemäßen Lipopeptide mit üblichen Träger- sowie gegebenenfalls Zusatz- und/oder Hilfstoffe in die bzw. eine geeignete Darreichungsform bringt.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### 1a) Herstellung einer Glycerinkultur von Actinoplanes sp. DSM 7358

100 ml Nährlösung (4 g/l Hefeextrakt, 15 g/l löslische Stärke, 1 g/l K₂HPO₄, 0,5 g/l MgSO₄ x 7 H₂O mit Wasser auf 1000 ml aufgefüllt, pH-Wert vor der Sterilisation 7,0) in einem sterilen 300 ml Erlenmeyerkolben werden mit dem Stamm Actinoplanes sp. DSM 7358 beimpft und 7 Tage bei 30°C und 150 UpM auf einer rotierenden Schüttelmaschine inkubiert. 1,5 ml dieser Kultur werden anschließend mit 2,5 ml 80 %igem Glycerin verdünnt und bei -20°C gelagert.

### 1b) Herstellung einer Kultur bzw. einer Vorkultur im Erlenmeyerkolben von Actinoplanes sp. DSM 7358:

Ein steriler 300 ml Erlenmeyerkolben mit 100 ml der folgende Nährlösung 30 g/l Saccharose, 2 g/l KNO₃, 1 g/l K₂HPO₄, 0,5 g/l MgSO₄ x 7 H₂O, 0,5 g/l KCl, 0,01 g/l FeSO₄ x 7 H₂O, 2 g/l Hefeextrakt, 5 g/l Pepton wird mit einer auf einem Schrägröhrchen (gleiche Nährlösung, jedoch mit 2 % Agar) gewachsenen Kultur oder mit 1 ml einer Glycerinkultur (s. Beispiel 1a) beimpft und auf einer Schüttelmaschine bei 180 UpM und 30°C inkubiert. Die maximale Produktion einer oder mehrerer Verbindungen der erfindungsgemäßen Lipopeptide ist nach ca. 120 Stunden erreicht. Zum Animpfen von 10 und 200 l Fermentern genügt eine 48 bis 96 Std. alte Submerskultur (Animpfmenge ca. 10 %) aus der gleichen Nährlösung.

### 2) Vergleichende Charakterisierung von Actinoplanes sp. DSM 7358

Zur Charakterisierung des Stammes Actinoplanes sp. DSM 7358 wird ein Vergleich mit nahe verwandten Stämmen nach der ISP-Methode nach Shirling und Gottlieb (Int. J. of Sys. Bacteriol. 16, 3 (1966) 313 bis 340) durchgeführt. Die Ergebnisse (siehe Tab. 1) zeigen, daß sich der Stamm Actinoplanes sp. DSM 7358 von den anderen Stämmen morphologisch und in seiner Physiologie unterscheidet.

**Tabelle 1:**

| | Actinoplanes sp. DSM 7358 | A.utahensis NRRL 12052 | A.brasiliensis ATCC 25844 | A.nipponensis ATCC 311455 |
|---|---|---|---|---|
| Luftmycel | - | - | + (ISP 3) | - |
| Sporangien | + | + | + | + |
| Medium | | | | |
| ISP 2 | orange | orange | gelborange | orange |
| ISP 3 | orange | orange | gelborange | orange |
| ISP 4 | orange | orange | gelborange | orange |
| ISP 5 | orange | orange | gelborange | orange |
| ISP 6 | orange | orange | gelborange | orange |
| | rotes | braunrotes | rotes | rotes |
| | Exopigment | Exopigment | Exopigment | Exopigment |
| Melanin | - | - | (+) | (+) |
| Glucose | + | + | + | + |
| Arabinose | + | + | + | + |
| Saccharose | + | + | + | + |
| Xylose | + | + | (+) | - |
| Inosit | + | + | (+) | (+) |
| Mannit | + | + | + | - |
| Fructose | + | + | + | - |
| Rhamnose | + | + | + | + |
| Raffinose | + | + | (+) | - |
| Cellulose | + | + | (+) | - |
| Melibiose | - | - | - | - |
| Amygdalin | - | + | + | + |
| Gelatine (Hydrolyse) | + | + | + | + |
| Citrat (Hydrolyse) | - | - | + | - |
| Harnstoff (hydrolyse) | + | - | - | - |
| Arginin hydrolase | + | - | - | - |
| ß-Galactosidase | - | - | - | - |
| Tryptophanase | - | - | - | - |
| Lysindecarboxylase | + | - | - | - |
| Acetoin (Bildung) | + | + | + | + |
| Indol (Bildung) | - | - | - | - |
| H₂S (Bildung) | - | - | - | - |
| NaCl Toleranz | 0 - 2,5 % | 0 - 2,5 % | 0 - 2,5 % | 0 - 2,5 % |

### 3a) Herstellung der Lipopeptide A 1437 B und D

Ein 500 1 Fermenter wird unter folgenden Bedingungen betrieben:
- Nährmedium:: 11 g/l Saccharose
6 g/l Fleischextrakt
0,3 g/l Hefeextrakt
0,6 g/l MgSO₄
0,1 g/l KH₂PO₄
10 µM FeCl₃ x 6H₂O
0,6 g/l L-Valin
pH 7,3 (vor der Sterilisation)
- Inkubationszeit:: 120 Stunden
- Inkubationstemperatur:: 30°C
- Rührergeschwindigkeit:: 50 UpM
- Belüftung:: 150 1 Min⁻¹

Durch wiederholte Zugabe von ethanolischer Polyollösung kann die Schaumbildung unterdrückt werden. Das Produktionsmaximum wird nach ca. 96 bis 120 Stunden erreicht.

Nach Beendigung der Fermentation von Actinoplanes sp. DSM 7358 wird die Kulturbrühe unter Zusatz von ca. 2 % Filterhilfsmittel (z.B. Celite^{®}) filtriert und das Kulturfiltrat auf 4°C gekühlt und ein pH von 1,5 eingestellt. Nach 4 Std. wird bei 10000 g zentrifugiert und der Niederschlag in Aqua dest. resuspendiert. Durch Neutralisieren der Suspension geht die genannte Substanz in Lösung. Diese wird eingefroren und lyophilisiert. Die Ausbeute beträgt ca. 1,5 g/l Rohprodukt (= 750 g).

### 3b) lonenchromatographische Auftrennung des Rohprodukts (enthält B + D)

Eine 3,2 l Chromatographie-Säule (10 cm ID x 40 cm H) wird mit DEAE-^{®}Sepharose Fast Flow gefüllt und mit 10 mM Kaliumphosphat-Puffer, pH 7,0 in 40 % Methanol (Puffer A) äquilibriert. Dann werden 25 g A 1437 B Rohprodukt (gewonnen nach Beispiel 3a), in 3,5 l Wasser gelöst, auf die Säule aufgetragen und mit 1 l Wasser, anschließend mit 6 l Puffer A gewaschen. Im Durchlauf und im Waschwasser befinden sich Verunreinigungen des Rohproduktes. Anschließend legt man einen 10 bis 100 mM Kaliumphosphat, pH 7,0-Gradienten in 40 % Methanol an. Mit 25 bis 35 mM Kaliumphosphat wird das A 1437 D-Peptid eluiert und mit 40 bis 55 mM Kaliumphosphat das Antibiotikum A 1437 B gewonnen. Die entsprechenden Fraktionen werden im Vakuum von Methanol befreit. Zur Entsalzung verwendet man eine 1 l fassende ®Dianion HP-20-Säule (Mitsubishi, Japan). Nun werden die 9 l reines B-Peptid beinhaltenden Fraktionen auf die Säule aufgetragen und anschließend mit 3 l entsalztem Wasser gewaschen. Eluiert wird mit Wasser-Isopropanol im Gradienten-Verfahren (0 bis 50 % Alkoholanteilen). Mit 15 bis 25 % Isopropanol wird das reine A 1437 B von dem Träger gewaschen. Dieser Säulenausfluß wird gesondert gesammelt, im Vakuum eingeengt und gefriergetrocknet. Es resultieren 4,8 g A 1437 B in 97 %iger Reinheit. Die entsprechende Entsalzung der A 1437 D-haltigen Fraktionen ergab 3,1 g des Antibiotikums. Reinheit 98 %.

### 4a) Herstellung der Lipopeptide A 1437 A und C

Die Herstellung erfolgt wie unter 3a beschrieben, lediglich das L-Valin wird im Produktionsmedium durch 4 g/l L-Leucin ersetzt und die Fermentation in einem 50 l Bioreaktor durchgeführt. Die Ausbeute beträgt 1,3 g/l Rohpeptid (= 65 g).

### 4b) Isolierung der Lipopeptide A 1437 A und C

10 g des Rohprodukts wurden in 100 ml aqua dest. gelöst und entsprechend dem unten stehenden Schema aufgearbeitet.

### Aufarbeitungsschema:

10 g Rohpeptid in 100 ml Aqua. dest.
↓
Adsorption an Q-Sepharose fast flow (Fa. Pharmacia)
(5 x 20 cm Säule)
↓
Elution mit folgendem Gradient:
Puffer A: NaH2PO4 1 mM in 50 % Methanol, pH 5,9
Puffer B: NaH2PO4 100 mM in 50 % Methanol, pH 5,3
20 Min.: Puffer A → in 60 Min auf 25 % Puffer B, weitere 30 Minuten bei 25 %
Puffer B;
↓
Lyophilisation der A 1437 A bzw. C Fraktionen und lösen in Laufmittel
↓
Entsalzung an Biogel P2 (100 - 200 mesh) der Fa. Biorad
(7 x 20 cm Säule)
↓ ↓
1,6 g Lipopeptid A 1437 A (80 %ig) 1,2 g Lipopeptid A 1437 C (80 %ig)
↓ ↓
RP-Chromatographie an Nucleosil® C18 (7 μm)
(20 x 250 mm Säule, 200 mg Auftrag)
Elution mit folgendem Gradient
Puffer A: Aqua bidest., 0,1 % TFA
Puffer B: Acetonitril
10 Min.: Puffer B → in 60 Min. auf 90 % Puffer B bei 10 ml/Minute Fluß
↓ ↓
Lyophilisation der A 1437 A bzw. C Fraktionen
↓ ↓
150 mg A 1437 A (Reinheit > 95 %ig) 154 mg A 1437 C (Reinheit > 95 %ig)

### 5a) Herstellung der Lipopeptide A 1437 E, F, G und H

Die Herstellung erfolgt wie in Beispiel 3a beschrieben, lediglich das L-Valin wird im Produktionsmedium durch 1,5 g/l L-Isoleucin ersetzt und die Fermentation in einem 50 l-Bioreaktor durchgeführt. Die Ausbeute beträgt 1,4 g/l Rohpeptid (70g).

### 5b) Ionenchromatographische Auftrennung des Rohpeptids

Auf der Säule, wie im Beispiel 3a beschrieben, werden 25 g Lipopeptid-Rohprodukt, gewonnen nach Beispiel 5a, entsprechend Beispiel 3a aufgetrennt. Mit
14 bis 19 mM Puffer wird das Lipopeptid A 1437 F (Ausbeute: 1,8 g),
18 bis 25 mM Puffer das A 1437 E (Ausbeute: 1,3g),
35 bis 50 mM Puffer das A 1437 H (Ausbeute: 2,7 g) und
64 bis 82 mM Puffer das A 1437 G (Ausbeute: 1,9 g) eluiert.

Die entsprechenden Fraktionen werden zusammengefasst und im Vakuum vom Methanol befreit.

### 5c) Aufreinigung der Komponenten aus Beispiel 5b auf Reverse Phase RP-18

Es wird eine 500 ml fassende präparative HPLC-Säule (5,1 cm (ID) x 25 cm H) mit ®LiChrosorbG RP-18, 10 µm, gefüllt und die salzhaltige A 1437 G beinhaltende Lösung mit 1,9 g des Antibiotikum aufgetragen. Eluiert wird im Gradientenverfahren mit 5 % Acetonitril in 10 mM Kaliumphosphatpuffer, pH 7,0 bis 36 % Acetonitril in 10 mM Kaliumphosphat-Puffer, pH 7,0. Mit 24 bis 26 % Acetonitril wird das Lipopeptid A 1437 G gewonnen. Nach dem Konzentrieren im Vakuum, Entsalzen auf 100 ml Adsorptionsharz ®Dianion HP-20 im Wasser/50 % Isopropanolsystem und Gefriertrocknung resultieren 1,1 g Lipopeptid A 1437 G in 99 %iger Reinheit.

Die entsprechende Nachreinigung des nach Beispiel 5b gewonnenen A 1437 H-Lösung geschieht mit dem Lösungsmittel-Gradienten 10 bis 50 % Acetonitril in 10 mM Kaliumphosphat-Puffer, pH 7,0. Die Elution des Antibiotikums erfolgt mit 37 bis 39 % Lösungsmittelanteilen. Konzentrierung der entsprechenden Fraktionen und Entsalzung auf ®Dianion HP-20 sowie Gefriertrocknung ergeben 2,2 g Lipopeptid A 1437 H in über 98 %iger Reinheit.

### 5d) Aufreinigung der Lipopeptid-Antibiotika A 1437 E und F aus Beispiel 5b auf MCI-Gel

Die nach Beispiel 5b gewonnene salzhaltige A 1437 F-Lösung mit 1,8 g des Antibiotikums wird auf 1 l MCI-Gel CHP 20P (Mitsubishi Kasei Corp.) aufgetragen. Die Säulenmaße betragen 6 cm ID x 35 cm H. Nach dem Beladen des Trägers mit dem zu trennenden Gut wird mit Puffer A (5 mM Kaliumphosphat-Puffer pH 7,0 mit 20 % Acetonitril) gewaschen und im Gradientenverfahren gegen den Puffer B (5 mM Kalium-Phosphat-Puffer, pH 7,0 mit 70 % Acetonitril) eluiert. 34 bis 35 % Lösungsmitttelanteile führen zur Elution des reinen Antibiotikums. Konzentrieren im Vakuum und Entsalzung auf ®Dianion HP-20 ergeben 1,4 g Lipopeptid A 1437 F in über 98 %iger Reinheit. Das analoge Vorgehen mit dem A 1437 E Rohprodukt des Beispiels 5a ergibt 1 g Lipopeptid A 1437 E in über 98 %iger Reinheit.

### 6a) Herstellung des Lipopeptides A 1437 K

Die Herstellung erfolgt wie unter 4a beschrieben, lediglich das L-Valin wird im Produktionsmedium durch 500 mg/l L-α-Aminobuttersäure (oder 1 g/l des Racemats) ersetzt und die Fermentation in einem 10 l Bioreaktor durchgeführt. Die Ausbeute beträgt 1,1 g/l Rohpeptid (= 10 g).

### 6b) Isolierung des Lipopeptids A 1437 K

10 g des Rohprodukts wurden in 100 ml aqua dest. gelöst und entsprechend dem unterstehenden Schema aufgearbeitet.

### Aufarbeitungsschema (A 1437 K)

- 10 g Rohpeptid in 100 ml aqua dest.
- Adsorption an Q-Sepharose fast flow (3,5 x 17 cm Säule)
- Elution mit folgendem Gradienten
   Puffer A: NaH₂PO₄ 1 mM in 50 % Methanol, pH 5,9
   Puffer B: NaH₂PO₄ 100 mM in 50 % Methanol, pH 5,3
   20 Min: Puffer A --→ in 45 Min. auf 25 % Puffer B, weitere 45 Min. bei
   25 % Puffer B
- Lyophilisation der A 1437 K Fraktionen
   Entsalzung an Biogel P2 (100-200 mesh)
   (7 x 20 cm Säule)
   700 mg A 1437 K (60 %ig)
- RP-Chromatographie an Nucleosil C₁₈ 7 µm (20 x 250 mm Säule)
   Auftrag: 50 mg des vorgereinigten Produkts
   Elution mit folgendem Gradient
   Puffer A: aqua bidest., 0,1 % TFA
   Puffer B: Acetonitril
   10 Min.: Puffer A / 5 % Puffer B -- in 60 Min. auf 70 % Puffer B bei
   10 ml/Minute Fluß
- Lyophilisation der A 1437 K Fraktionen
   5,6 mg A 1437 K (> 95 %ig)

### 7a) Herstellung der Lipopeptide A 1437 L und M

Die Herstellung erfolgt wie unter 4a beschrieben, lediglich das L-Valin wird im Produktionsgemisch durch 500 mg/l L-Norvalin (oder 1 g/l des Racemats) ersetzt und die Fermentation in einem 10 l Bioreaktor durchgeführt. Die Ausbeute beträgt 1,2 g/l Rohpeptid (= 12g).

### 7b) Isolierung der Lipopeptide A 1437 L und M

10 g des Rohprodukts werden in 100 ml aqua dest gelöst und entsprechend dem untenstehenden Schema aufgearbeitet.

### Aufarbeitungsschema:

- 10 g Rohpeptid in 100 ml aqua dest.
- Adsorption an Q-Sepharose fast flow
   (3,5 x 17 cm Säule)
- Elution mit folgendem Gradient
   Puffer A: NaH₂PO₄ 1 mM in 50 % Methanol, pH 5,9
   Puffer B: NaH₂PO₄ 100 mM in 50 % Methanol, pH 5,3
   20 Min: Puffer A --- in 45 Min. auf 25 % Puffer B, weitere 45 Min. bei
   25 % Puffer B
- Lyophilisation der A 1437 L und M Fraktionen
- Entsalzung an Biogel P2 (100-200 mesh)
   (7 x 20 cm Säule)
   900 mg A 1437 L und M (ca. 60 %ig)
- RP-Chromatographie an Nucleosil C₁₈ 7 µm (20 x 250 mm Säule)
   Auftrag: 50 mg des vorgereinigten Produkts
   Elution mit folgendem Gradient
   Puffer A: aqua bidest., 0,1 % TFA
   Puffer B: Acetonitril
   10 Min.: Puffer A / 5 % Puffer B --→ in 60 Min. auf 70 % Puffer B bei
   10 ml/Minute Fluß
- Lyophilisation der A 1437 L und M Fraktionen
   5,8 mg A 1437 L (> 95 %ig) 6,7 mg A 1437 M ( > 95 %ig)

### 8) HPLC System zum Nachweis der A 1437 Lipopeptide

Das unten beschriebene System erlaubt die Trennung und Quantifiziertung der Lipopeptide im Rohgemisch bzw. im Kulturfiltrat; die Retentionszeiten liegen zwischen 11,5 Minuten (A 1437 E) und ca. 15,9 Minuten (A 1437 H).

| | | |
|---|---|---|
| Laufmittel | A | Kalium-Phosphatpuffer pH 7,0, 10 mM |
| | B | Acetonitril |

| Gradient | t[Min] | Fluß[ml/min] | A[%] | B[%] |
|---|---|---|---|---|
| | 0 | 1,5 | 80 | 20 |
| | 15 | 1,5 | 50 | 50 |
| | 15,5 | 2 | 00 | 100 |
| | 16,5 | 2 | 00 | 100 |
| | 17 | 1,5 | 80 | 20 |
| | 22 | 1,5 | 80 | 20 |

- Säule:: Shandon ODS Hypersil RP 18 (120 x 4,6 mm mit 20 x 4,6 mm Vorsäule) oder
Nucleosil 120 RP 18 (120 x 4,6 mm mit 20 x 4,6 mm Vorsäule)
- Fluß:: 1,5 ml/min
- Detektion:: 210 nm
- Injektion:: 10 µl

### 9) Vergleich zwischen Actinoplanes nipponensis ATCC 31145 und Actinoplanes spec. DSM 7358

Von beiden Stämmen wird zunächst wie in Beispiel 1b beschrieben eine Vorkultur angezogen und diese zum Beimpfen folgender Produktionsmedien verwendet.
- Medium 1:: wie in Beispiel 3a beschrieben
- Medium 2:: wie Medium 1 jedoch ohne L-Valin
- Medium 3:: Glucose 30 g/l; Sojamehl 20 g/l; Fe₂(SO₄)₃ 0,3 g/l; MnCl₂x4H₂O 0,3 g/l und CoCl₂x6 H₂O pH 7,3
jeweils 100 ml Medium in einem 300 ml Erlenmeyerkolben.

Die Inkubation erfolgt bei 30°C auf einer rotierenden Schüttelmaschine. Nach 48, 96 und 144 Stunden wird die Konzentration der A 1437 Lipopeptide im Kulturfiltrat mittels HPLC (s. Bsp. 8) bestimmt. In Medium 2 und Medium 3 ist bei dem Stamm Actinoplanes nipponensis ATCC 31145 keinerlei Lipopeptid nachweisbar. In Medium 1 können nach 144 Stunden einige Peptide in sehr kleinen Mengen nachgewiesen werden. Legt man eine identische spez. Extinktion dieser Verbindungen im Vergleich zu den A 1437 Peptiden zugrunde, so liegt die gebildete Menge um mindestens den Faktor 100 (< 1 mg/l) unter den Konzentration von A 1437 B, wie sie vom Stamm Actinoplanes spec. DSM 7358 in Medium 1 nach der gleichen Zeit synthetisiert wird.

### 10) Wirkung der A 1437 Lipopeptide

Die Empfindlichkeit relevanter Keime gegen die A 1437 Lipopeptide wird mittels eines Agar-Verdünnungs-Tests bestimmt. Als Agar wird Müller-Hinton-Agar, dem im Fall von S. pyogenes und der Enterococcen 10 % Pferdeblut zugesetzt wird, verwendet. Die antibiotikahaltigen Platten werden mit einem Mehrkanallmpfgerät beimpft (5 x 10⁴ cfu/Impfstelle, einer stationären Kultur des jeweiligen Stammes). Die MHK-Werte (Minimale Hemmkonzentration) werden bei 37°C abgelesen. Als MHK-Wert wird die Konzentration des Antibiotikums definiert, bei der nach 24 Stunden Inkubation kein sichtbares Wachstum der Keime detektierbar ist.
Die Ergebnisse sind in Tabelle 2 zusammengefaßt. Das als Kontrolle verwendete Amphomycin wurde von Boehringer Mannheim (Deutschland) bezogen. Amphomycin ist dort als Feinchemikalie erhältlich.

**Tabelle 2:**

| A 1437 Substanzen: In vitro Aktivität (AB-Spektrum); Konzentration in µg/ml | | | | | | |
|---|---|---|---|---|---|---|
| A 1437 A | 0.391 | 0.781 | 0.391 | 1.56 | 0.391 | 3.13 |
| A 1437 B | 0.098 | 0.195 | 0.098 | 0.195 | 0.049 | 0.391 |
| A 1437 C | 0.195 | 0.781 | 0.391 | 3.13 | 0.781 | 3.13 |
| A 1437 D | 0.049 | 0.195 | 0.049 | 0.781 | 0.391 | 0.781 |
| A 1437 E | 0.098 | 0.195 | 0.094 | 0.098 | 0.025 | 0.195 |
| A 1437 F | 0.098 | 0.391 | 0.195 | 1.56 | 0.781 | 1.56 |
| A 1437 G | 0.098 | 0.195 | 0.049 | 0.088 | 0.025 | 0.195 |
| A 1437 H | 0.049 | 0.098 | 0.049 | 0.195 | 0.049 | 0.195 |
| Amphomycin | 0.195 | 0.781 | 0.391 | 1.56 | 0.391 | 1.56 |

| A 1437 Substanzen: In vitro Aktivität (Vancomycin-resistente Stämme); Konzentration in µg/ml | | | |
|---|---|---|---|
| | Enterococcus faecium VR1 | Enterococcus faecium VR2 | Streptococcus pyogenes |
| A 1437 A | nicht bestimmt | | |
| A 1437 B | 1 | 1 | 0.5 |
| A 1437 C | 4 | 4 | 4 |
| A 1437 D | 2 | 2 | 1 |
| A 1437 E | 4 | 4 | 1 |
| A 1437 F | 4 | 4 | 1 |
| A 1437 G | 0.25 | 0.25 | 0.125 |
| A 1437 H | 1 | 1 | 0.5 |
| Amphomycin | 4 | 4 | 2 |

Die Verbindungen K, L, M besitzen eine mit den Verbindungen A-H vergleichbare in vitro Aktivität.

### Beispiel 11 a: Charakterisierung von A 1437 D

Das Lipopeptid A 1437 D wird als amorpher Feststoff isoliert
Drehwert: +35° (c = 0,1; Methanol)
HPLC: Retentionszeit: 15,1 Minuten
- Aminosäuren:: 2 Asparaginsäuren
1 Asparagin
1 β-Methylaspartat
2 Glycin
2 2,3-Diaminobuttersäure
1 Prolin
1 Pipecolinsäure
1 Valin
FAB-MS: m/e = 1303, 6952 [(M+H)⁺]
Molmasse: 1302, 6884 (C₅₉H₉₄N₁₄O₁₉)
CID-MS: m/z = 356, 491, 517, 520, 741, 761, 938, 982
IR (KBr): *v* = 3420 (br) cm⁻¹, 2930, 1660, 1530, 1450, 1400.

### Beispiel 11b: Charakterisierung von A 1437 B

Das Lipopeptid A 1437 B wird als amorpher Feststoff isoliert
Drehwert: + 27° (c = 0,1; Methanol)
HPLC: Retentionszeit: 12,8 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
FAB-MS: m/e = [(M+H)⁺]
Molmasse: 1303 (C₅₉H₉₃N₁₃O₂₀)
CID-MS: m/z = 356, 407, 518, 521, 741, 762, 938, 982
IR (KBr): v = 3420 (br) cm⁻¹, 2925, 1650, 1535, 1450, 1400.

### Beispiel 11 c: Charakterisierung von A 1437 C

Das Lipopeptid A 1437 C wird als amorpher Feststoff isoliert
Drehwert: +30° (c = 0,1; Methanol)
HPLC: Retentionszeit: 14,1 Minuten
- Aminosäuren:: 2 Asparaginsäuren
1 Asparagin
1 β-Methylaspartat
2 Glycin
2 2,3-Diaminobuttersäure
1 Prolin
1 Pipecolinsäure
1 Valin
Molmasse: 1288 (C₅₈H₉₂N₁₄O₁₉)
CID-MS: m/z = 356, 392, 503, 503, 741, 747, 938, 981
IR (KBr): *v* = 3420 (br) cm⁻¹, 2930, 1660, 1530, 1450, 1400.

### Beispiel 11 d: Charakterisierung von A 1437 A

Das Lipopeptid A 1437 A wird als amorpher Feststoff isoliert
Drehwert: +30° (c = 0,1; Methanol)
HPLC: Retentionszeit: 11,8 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
Molmasse: 1289 (C₅₈H₉₁N₁₃O₂₀)
CID-MS: m/z = 356,478, 504, 507, 741, 748, 938, 981
IR (KBr): *v* = 3420 (br) cm⁻¹, 2925, 1650, 1535, 1450, 1400.

### Beispiel 11e: Charakterisierung von A 1437 F

Das Lipopeptid A 1437 F wird als amorpher Feststoff isoliert
Drehwert: +31° (c = 0,1; Methanol)
HPLC: Retentionszeit: 13,8 Minuten
- Aminosäuren:: 2 Asparaginsäuren
1 Asparagin
1 β-Methylaspartat
2 Glycin
2 2,3-Diaminobuttersäure
1 Prolin
1 Pipecolinsäure
1 Valin
Molmasse: 1288 (C₅₈H₉₂N₁₄O₁₉)
CID-MS: m/z = 356, 392, 503, 506, 741, 747, 938, 981
IR (KBr): *v* = 3420 (br) cm⁻¹, 2930, 1660, 1530, 1450, 1400.

### Beispiel 11f: Charakterisierung von A 1437 E

Das Lipopeptid A 1437 E wird als amorpher Feststoff isoliert
HPLC: Retentionszeit: 11,5 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
Molmasse: 1289 (C₅₈H₉₁N₁₃O₂₀)
CID-MS: m/z = 356, 393, 504, 507, 741, 748, 938, 981
IR (KBr): *v* = 3420 (br) cm⁻¹, 2925, 1650, 1535, 1450, 1400.

### Beispiel 11g: Charakterisierung von A 1437 H

Das Lipopeptid A 1437 H wird als amorpher Feststoff isoliert
Drehwert: + 32° (c = 0,1; Methanol)
HPLC: Retentionszeit: 15,9 Minuten
- Aminosäuren:: 2 Asparaginsäuren
1 Asparagin
1 β-Methylaspartat
2 Glycin
2 2,3-Diaminobuttersäure
1 Prolin
1 Pipecolinsäure
1 Valin
Molmasse: 1316 (C₆₀H₉₆N₁₄O₁₉)
CID-MS: m/z = 356, 420, 531, 534, 741, 775, 938, 981
IR (KBr): *v* = 3420 (br) cm⁻¹, 2930, 1660, 1530, 1450, 1400.

### Beispiel 11h: Charakterisierung von A 1437 G

Das Lipopeptid A 1437 G wird als amorpher Feststoff isoliert
Drehwert: + 34° (c = 0,1; Methanol)
HPLC: Retentionszeit: 13,6 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
Molmasse: 1317 (C₆₀H₉₅N₁₃O₂₀)
CID-MS: m/z = 356, 421, 532, 535, 741, 776, 938, 981
IR (KBr): *v* = 3420 (br) cm⁻¹, 2925, 1650, 1535, 1450, 1400.

### Beispiel 11i: Charakterisierung von A 1437 K

Das Lipopeptid A 1437 K wird als amorpher Feststoff isoliert
HPLC: Retentionszeit: 12,5 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
FAB-MS: m/e = [(M+H)⁺]
Molmasse: 1299 (C₅₈H₉₁N₁₃O₂₀)
CID-MS: m/z = 393, 504, 507, 741, 748, 938, 981
IR (KBr): *v* = 3420 (br) cm⁻¹, 2925, 1650, 1535, 1450, 1400.

### Beispiel 11j: Charakterisierung von A 1437 L

Das Lipopeptid A 1437 L wird als amorpher Feststoff isoliert
HPLC: Retentionszeit: 13,0 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
FAB-MS: m/e = [(M+H]⁺]
Molmasse: 1289 (C₅₉H₉₃N₁₃O₂₀)
CID-MS: m/z = 407, 518, 741, 761, 938, 981
IR (KBr): *v* = 3420 (br) cm⁻¹, 2925, 1650, 1535, 1450, 1400.

### Beispiel 11k: Charakterisierung von A 1437 M

Das Lipopeptid A 1437 M wird als amorpher Feststoff isoliert
HPLC: Retentionszeit: 9,8 Minuten
- Aminosäuren:: 3 Asparaginsäuren
1 β-Methylaspartat
2 Glycin
2 2,3-Diaminobuttersäure
1 Prolin
1 Pipecolinsäure
1 Valin
FAB-MS: m/e = [(M+H)⁺]
Molmasse: 1275 (C₅₇H₈₉N₁₃O₂₀)
CID-MS: m/z = 379, 490, 493, 724, 741, 938, 981
IR (KBr): *v* = 3420 (br) cm⁻¹, 2925, 1650, 1535, 1450, 1400.

### Beispiel 12: C13-chem. Verschiebungen

Die Tabelle zeigt die C13-chem. Verschiebungen der CH-Signale von A 1437B

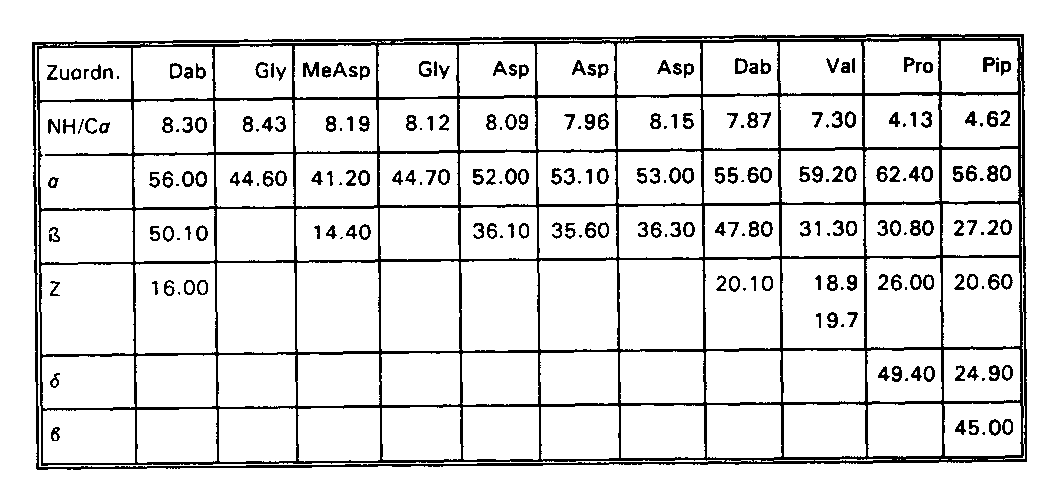

Um einen Vergleich mit den IH-Daten zu ermöglichen, wurden die NH-chem.-Verschiebungen der NH-Signale bzw. für Pip und Pro die CαH-Verschiebungen der entsprechenden Spinsysteme mit angegeben.

## Patentansprüche

1. Lipopeptide der Formel I, 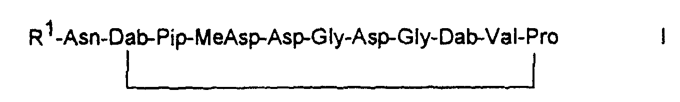
R1-Asn-Dab-Pip-MeAsp-Asp-Gly-Asp-Gly-Dab-Val-Pro I
 in der
R¹ eine einfach oder mehrfach ungesättigte, gesättigte oder unabhängig davon eine verzweigte oder unverzweigte Fettsäure oder Hydroxyfettsäure mit einer Kettenlänge von 6 bis einschließlich 22 Kohlenstoffatomen bedeutet.

2. Lipopeptid nach Anspruch 1, dadurch gekennzeichnet, daß R¹ eine Kettenlänge von 10 bis einschließlich 20 Kohlenstoffatomen aufweist.

3. Lipopeptid nach Anspruch 1, dadurch gekennzeichnet, daß R¹ eine Kettenlänge von 12,13,14 oder 15 Kohlenstoffatomen aufweist.

4. Lipopeptid nach Anspruch 1, dadurch gekennzeichnet, daß R¹ eine
a) iC₁₃-Fettsäure,
b) iC₁₄-Fettsäure,
c) aiC₁₃-Fettsäure oder eine
d) aiC₁₅-Fettsäure bedeutet.

5. Lipopeptid nach Anspruch 4, dadurch gekennzeichnet, daß
R₁ = 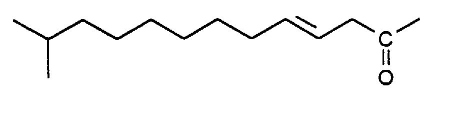 bedeutet.

6. Lipopeptid nach Anspruch 4, dadurch gekennzeichnet, daß
R₁ = 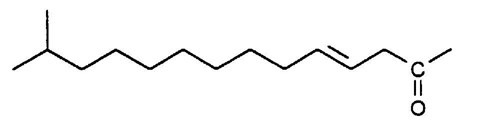 bedeutet.

7. Lipopeptid nach Anspruch 4, dadurch gekennzeichnet, daß
R₁ = 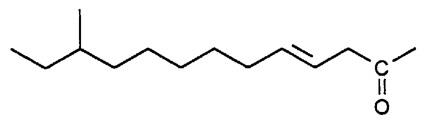 bedeutet.

8. Lipopeptid nach Anspruch 4, dadurch gekennzeichnet, daß
R₁ = 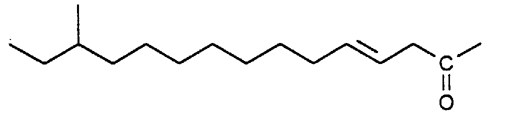 bedeutet.

9. Verfahren zur Herstellung eines Lipopeptides der Formel I gemäß einem oder mehreren der Ansprüche 1 bis 8, dadurch gekennzeichnet, daß man Actinoplanes sp. in einem Kulturmedium fermentiert, bis sich ein oder mehrere Lipopeptide in dem Kulturmedium anhäufen und man das Lipopeptid der Formel I aus dem Kulturmedium aufreinigt.

10. Verfahren nach Anspruch 9, dadurch gekennzeichnet, daß die Aufreinigung erfolgt, indem man die Lipopeptide aus dem Kulturmedium unter Verwendung von Säuren bei pH 0,5 bis einschließlich pH 4 fällt, man dann gegebenenfalls die aus der Fällung erhaltenen Lipopeptide durch Chromatographie an Anionenaustauschern oder Chromatographie an hydrophober Matrix aufreinigt, wobei die beiden Chromatographien alternativ oder nachfolgend in beliebiger Reihenfolge durchgeführt werden können.

11. Verfahren nach Anspruch 9 oder 10, dadurch gekennzeichnet, daß man Actinoplanes sp. DSM 7358 fermentiert.

12. Arzneimittel, enthaltend ein oder mehrere Lipopeptide der Formel I gemäß einem oder mehreren der Ansprüche 1 bis 8 und gegebenenfalls pharmazeutische Träger.

13. Verwendung eines Lipopeptids der Formel I gemäß einem der Ansprüche 1 bis 8 zur Herstellung eines Antibiotikums gegen gram-positive Bakterien.

14. Verwendung nach Anspruch 13, dadurch gekennzeichnet, daß die Bakterien glycopeptidresistente Bakterien sind.

15. Actinoplanes sp. DSM 7358.

## Claims

1. Lipopeptides of the formula I 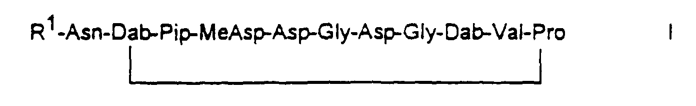
R1-Asn-Dab-Pip-MeAsp-Asp-Gly-Asp-Gly-Dab-Val-Pro I
 in which
R¹ is a singly or multiply unsaturated, saturated or, independently thereof, a branched or unbranched fatty acid or hydroxy fatty acid with a chain length of from 6 to 22, inclusive, carbon atoms.

2. A lipopeptide as claimed in claim 1, wherein R¹ has a chain length of from 10 to 20, inclusive, carbon atoms.

3. A lipopeptide as claimed in claim 1, wherein R¹ has a chain length of 12, 13, 14 or 15 carbon atoms.

4. A lipopeptide as claimed in claim 1, wherein R¹ is an
a) iC₁₃-fatty acid,
b) iC₁₄-fatty acid,
c) aiC₁₃-fatty acid or a
d) aiC₁₅-fatty acid,

5. A lipopeptide as claimed in claim 4, wherein
R¹ =

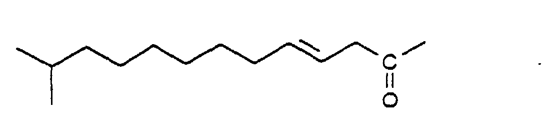

6. A lipopeptide as claimed in claim 4, wherein
R¹ =

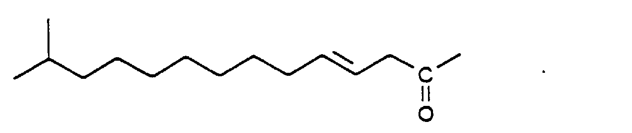

7. A lipopeptide as claimed in claim 4, wherein
R¹ =

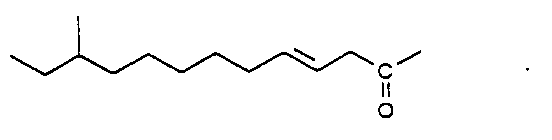

8. A lipopeptide as claimed in claim 4, wherein
R¹ =

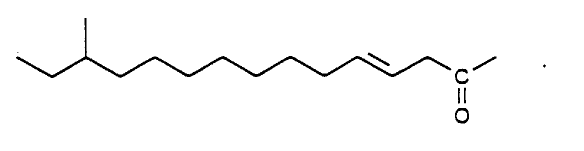

9. A process for the production of a lipopeptide of the formula I as claimed in one or more of claims 1 to 8, which comprises fermenting Actinoplanes sp. in a culture medium until one or more lipopeptides accumulate in the culture medium and purifying the lipopeptide of the formula I from the culture medium.

10. The process as claimed in claim 9, wherein the purification takes place by precipitating the lipopeptides from the culture medium using acids at pH 0.5 to pH; 4, inclusive, then where appropriate purifying the lipopeptides obtained from the precipitation by chromatography on anion exchangers or chromatographic on a hydrophobic matrix, it being possible for the to chromatographies to be carried out as alternatives or consecutively in any desired sequence.

11. The process as claimed in claim 9 or 10, wherein Actinoplanes sp. DSM 7358 is fermented.

12. A pharmaceutical containing one or more lipopeptides of the formula I as claimed in one or more of claims 1 to 8 and, where appropriate, pharmaceutical vehicles.

13. The use of a lipopeptide of the formula I as claimed in any one of claims 1 to 8 for the production of an antibiotic against Gram-positive bacteria.

14. The use as claimed in claim 13, wherein the bacteria are glycopeptide-resistant bacteria.

15. Actinoplanes sp. DSM 7358.

## Revendications

1. Lipopeptides de formule I 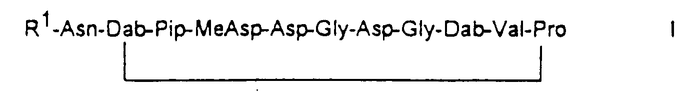
R1-Asn-Dab-Pip-MeAsp-Asp-Gly-Asp-Gly-Dab-Val-Pro I
 dans laquelle
R¹ représente un acide gras ou acide gras hydroxylé, saturé, une ou plusieurs fois insaturé, ou, indépendamment de cela, ramifié ou non ramifié, ayant une longueur de chaîne de 6 à y compris 22 atomes de carbone.

2. Lipopeptide selon la revendication 1, caractérisé en ce que R¹ présente une longueur de chaîne de 10 à y compris 20 atomes de carbone.

3. Lipopeptide selon la revendication 1, caractérisé en ce que R¹ présente une longueur de chaîne de 12, 13, 14 ou 15 atomes de carbone.

4. Lipopeptide selon la revendication 1, caractérisé en ce que R¹ représente
a) un acide gras iso en C₁₃,
b) un acide gras iso en C₁₄,
c) un acide gras ante-iso en C₁₃ ou
d) un acide gras ante-iso en C₁₅.

5. Lipopeptide selon la revendication 4, caractérisé en ce que
R₁ représente

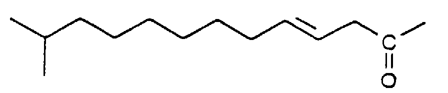

6. Lipopeptide selon la revendication 4, caractérisé en ce que
R₁ représente

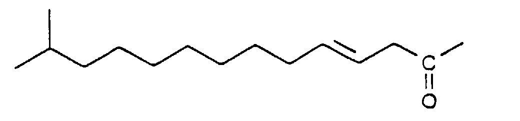

7. Lipopeptide selon la revendication 4, caractérisé en ce que
R₁ représente

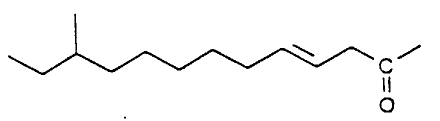

8. Lipopeptide selon la revendication 4, caractérisé en ce que
R₁ représente

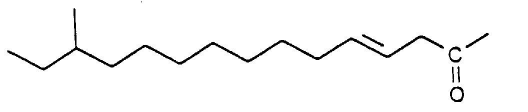

9. Procédé pour la production d'un lipopeptide de formule I selon une ou plusieurs des revendications 1 à 8, caractérisé en ce que l'on cultive par fermentation *Actinoplanes sp.* dans un milieu de culture, jusqu'à ce qu'un ou plusieurs lipopeptides s'accumulent dans le milieu de culture, et on purifie le lipopeptide de formule I à partir du milieu de culture.

10. Procédé selon la revendication 9, caractérisé en ce que l'on effectue la purification en faisant précipiter les lipopeptides dans le milieu de culture, à un pH de 0,5 à y compris 4, à l'aide d'acides, puis en purifiant éventuellement les lipopeptides, obtenus après la précipitation, par chromatographie sur des échangeurs d'anions ou chromatographie sur matrice hydrophobe, les deux chromatographies pouvant être effectuées l'une ou l'autre ou successivement en un ordre quelconque.

11. Procédé selon la revendication 9 ou 10, caractérisé en ce que l'on cultive par fermentation *Actinoplanes sp.* DSM 7358.

12. Médicament, contenant un ou plusieurs lipopeptides de formule I selon une ou plusieurs des revendications 1 à 8, et éventuellement des véhicules pharmaceutiques.

13. Utilisation d'un lipopeptide de formule I selon l'une des revendications 1 à 8, pour la fabrication d'un antibiotique contre des bactéries à Gram positif.

14. Utilisation selon la revendication 13, caractérisée en ce que les bactéries sont des bactéries résistantes aux glycopeptides.

15. *Actinoplanes sp.* DSM 7358.
